**Europäisches Patentamt**

**European Patent Office**

**Office européen des brevets**

(19)

(11) Publication number : **0 514 182 A1**

(12) **EUROPEAN PATENT APPLICATION**

(21) Application number : **92304385.5**

(22) Date of filing : **14.05.92**

(51) Int. Cl.⁵ : **A61B 5/117**

(30) Priority : **15.05.91 GB 9110517**

(43) Date of publication of application :
**19.11.92 Bulletin 92/47**

(84) Designated Contracting States :
**DE FR GB NL**

(71) Applicant : **THORN EMI plc**
**4 Tenterden Street**
**London W1A 2AY (GB)**

(72) Inventor : **Jackson, Michael**
**11 The Green, Hayes**
**Middlesex, UB3 2RW (GB)**

(74) Representative : **Hurst, Richard Arthur**
**Alexander et al**
**THORN EMI Patents Limited Central Research**
**Laboratories Dawley Road**
**Hayes, Middlesex UB3 1HH (GB)**

(54) **Apparatus for imaging a fingerprint.**

(57)   A fingerprint (2) to be imaged is brought into contact with an elastomeric membrane (6) which conforms with the ridges and throughs of the fingerprint (2). The membrane (6) is urged into contact with the hypotenuse (5) of a prism (4) thereby to interrupt the total internal reflection of light occuring at the hypotenuse (5). The interrupted total internal reflection occurs only at those points where the membrane (6) overlies the ridges of fingerprint (2).

*Fig.4.*

The present invention relates to an apparatus for imaging a fingerprint.

Methods and apparatus for characterising images of fingerprints are known and generally require an image of a fingerprint to be formed either electronically or physically, this formed image being the basis of the characterisatiion technique. In order for the characterisation to be effective and as accurate as possible, it is a requirement that the image of the fingerprint so formed be as sharp as possible and that there be high contrast as between the ridges and recesses present in the actual fingerprint.

One method of obtaining such fingerprint images is known as Frustrated Total Internal Reflection, such as is described in the paper "Fingerprint imagery using frustrated total internal reflection" by LA Gerbardt, JB Attili, DH Crockett and AM Presler of Rensselaer Polytechnic Institute given at the 1986 International Carnahan Conference on Security Technology, Gothenburg, Sweden, August 12-14 1986.

The basic principle of frustrated total internal reflection is that light, if incident on an interface going from a higher refractive index to a lower refractive index (e.g. glass to air) will be totally reflected if the incident angle is large enough. However, if material (such as a finger) with a relatively high refractive index comes into contact with the interface from the side of lower refractive index, then the conditions which cause total internal reflection will be disturbed and some or all of the light is transmitted through the interface into the contacting material, where it becomes absorbed, scattered or refracted. The totally internally reflected light is then caused to be incident on an external, imaging device, such as an electronic CCD camera.

Generally, however, such systems have been found to produce high-contrast, good definition images of a fingerprint only when the fingerprint is held in contact with the interface for at least several seconds. If such a system is to be employed in a security or an identification environment, then such a delay would be intolerable.

Another method for achieving images of a fingerprint is disclosed in US Patent number 4,360,300. This describes a system comprising an optical element which is to some extent deformable in response to finger pressure, thereby to reflect or scatter the incident light in a different direction to that which it would follow if it were totally internally reflected, hence enabling formation of the fingerprint image using a CCD camera or similar device. The element is a sensor plate composed of a transparent polymer which is elastic so as to form a latent topographic relief of a fingerprint pattern while exposed to the fingerprint. Light is passed into the sensor plate and becomes totally internally reflected in those positions which are not locally disturbed or influenced by contact with the friction ridges of the contacting finger.

However, such a system suffers from the disadvantage that even though the fingerprint is in direct contact with the sensor plate, thereby forming a true latent topographical relief of the fingerprint pattern which itself is unaffected by dirt or grease from the fingerprint, deposits of dirt or grease will accumulate on the surface of the sensor plate. It will be appreciated that because it is the sensor plate itself which directly causes the total internal reflection, these deposits will optically couple to the surface of the sensor plate, absorbing and scattering the light, and this results in lower contrast, blurred images.

It is thus as object of the present invention to provide apparatus for obtaining an image of a fingerprint, wherein the problems associated with build-up of grease and dirt upon an imaging surface are at least alleviated.

Thus, according to the present invention there is provided apparatus for imaging a fingerprint comprising: a light transmitting member having a first major surface at which light transmitted through the light transmitting member may undergo total internal reflection; and a light-absorbing elastic member being arranged adjacent to and spaced from the first major surface for enabling the elastic member, when urged into contact with the light transmitting member by a fingerprint, to conform sympathetically with the fingerprint such that only selected areas of the elastic member, corresponding to fingerprint ridges, contact the first major surface.

Hence by utilising a light-absorbing elastic member which has a normal or rest position spaced from the light transmitting member, and which, when pressed towards the light transmitting member by a fingerprint only contacts the light transmitting member in those positions where there is a fingerprint ridge, a clear sharp contrast fingerprint image is obtained. Furthermore, since the finger which is to be imaged is never in direct contact with the light transmitting member, then the above problems associated with the build-up of dirt and/or grease on the imaging surface are obviated.

Preferably the thickness of the elastic member is less than the inter-ridge spacing of the fingerprint for imaging. Thus, the elastic member may conform with the fingerprint to such an extent that, when urged into contact with the light transmitting member, a clear contour distinction of the elastic member is obtained as between those areas corresponding with the fingerprint ridges, and those conforming with the fingerprint troughs.

Preferably, the elastic member comprises an elastomer.

Additionally, or alternatively, the elastic member may be black in colour thereby to enhance the image contrast.

The invention will now be described, by way of example only, with reference to the following drawings of which:

Figure 1 illustrates schematically an example of a prior art fingerprint imaging apparatus;

Figure 2 illustrates schematically the plan view arrangement of the elastic member;

Figure 3 illustrates schematically a portion of fingerprint imaging apparatus including the elastic member of the present invention;

Figure 4 illustrates schematically the operation of a fingerprint imaging apparatus in accordance with the present invention; and

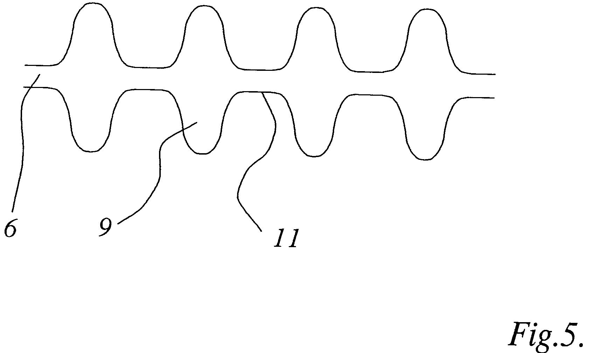

Figure 5 illustrates schematically a sectional view of an alternative form of elastic member.

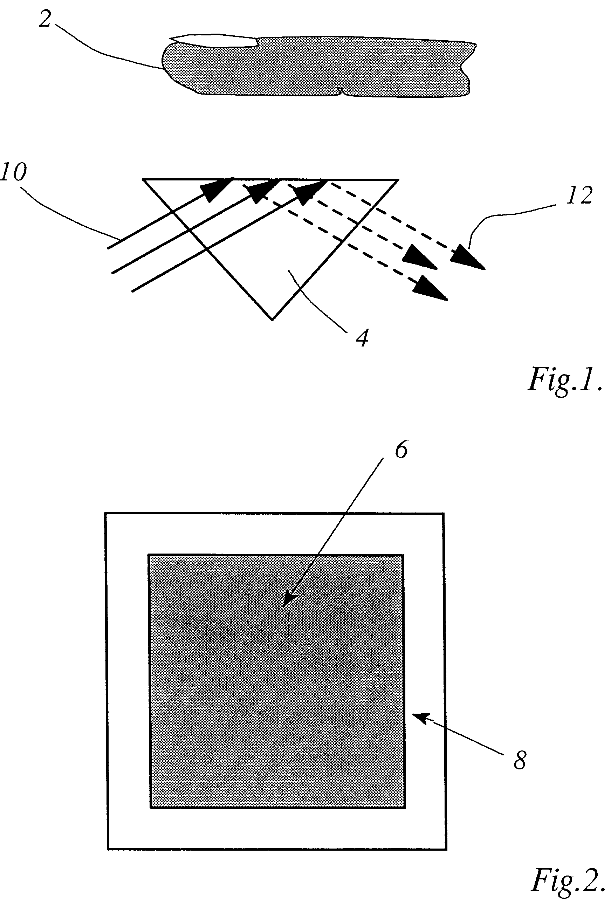

Referring firstly to Figure 1 in which is schematically illustrated one example of a prior art frustrated total internal reflection fingerprint imaging apparatus, it will be seen that a fingerprint 2 must be brought into direct contact with a prism 4 in order for frustrated total internal reflection to occur. As has been detailed above, such a device will generally accumulate dirt and grease at the point at which the fingerprint 2 contacts prism 4 thereby leading to the formation of images with poor contrast and quality.

By referring now to Figure 2 it will be seen that an elastic member in the form of an elastomeric membrane 6 has been stretched over a supporting frame 8. The membrane 6 and frame 8 form a "finger guide" which defines where the user of the apparatus must position the fingerprint in order to obtain an image thereof.

Because frustrated total internal reflection relies upon clear differences as between the points of contact and non-contact with an imaging surface, that is to say the fingerprint ridges and troughs respectively, the membrane 6 is to conform to the fingerprint 2 contour profile as accurately as possible. Thus, it is desirable that the thickness of the membrane 6 be less than the inter-ridge spacing of the fingerprint 2. In the present example, the thickness of the membrane 6 when stretched over the frame 8 is substantially uniform over its area and is 0.05 mm, whereas the average inter-ridge spacing of a fingerprint is approximately 0.5 mm. Hence membrane 6 is able to conform very closely to the profile of the fingerprint 2. The advantage this effect offers will be explained hereafter.

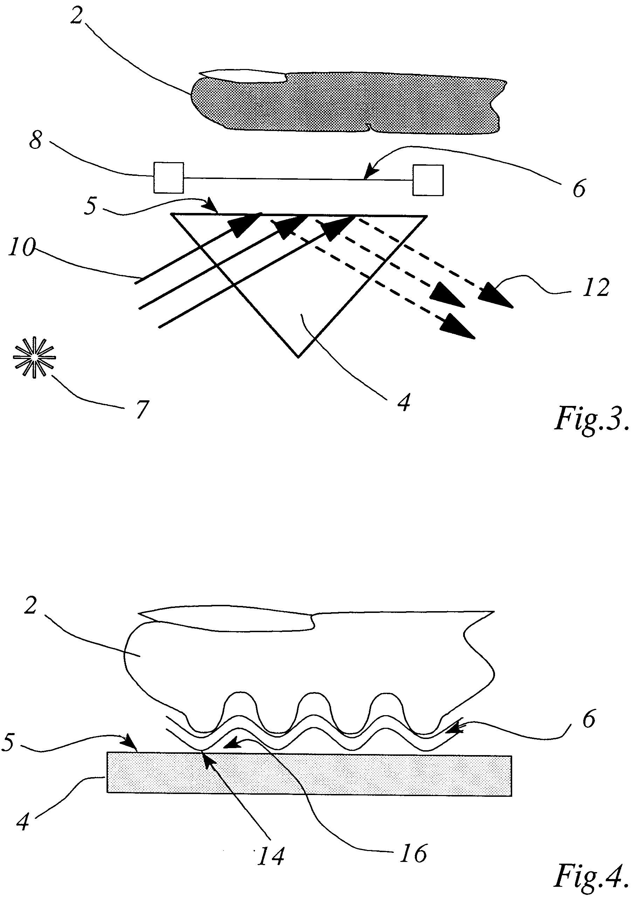

Referring now also to Figure 3, in which similar components to those of Figure 1 have been similarly numbered, it will be seen that membrane 6 mounted on frame 8 has been placed between the fingerprint 2 and a light transmitting member in the form of a prism 4. It is convenient to utilise a first major surface, the hypotenuse 5, of the prism 4 for fingerprint imaging.

Figure 3 illustrates the effect of (non-frustrated) total internal reflection within prism 4. Incident light 10 from light source 7 which strikes the hypotenuse at an angle greater than the critical angle will be totally internally reflected and exit prism 4 as reflected light 12.

It will be appreciated by those skilled in the art, however, that at those points of the hypotenuse at which membrane 6 is caused by the ridges of fingerprint 2 to contact the prism 4, the total internal reflection will be frustrated. The effect of this is that those corresponding regions of the reflected light 12 are themselves frustrated and hence an image of the fingerprint may be formed arising from the contrast between the frustrated and non-frustrated reflected regions of light 12.

By reference now also to Figure 4, the frustrated total internal reflection caused by membrane 6 will be further explained. Those skilled in the art will appreciate the shortcomings associated with prior art fingerprint imaging apparatus in which a fingerprint is brought into direct contact with the imaging surface. As well as the problems of grease and dirt on the surface, such systems provide images of varying degrees of quality according to the degree of optical coupling between the skin and imaging surfaces. For instance a wet fingerprint often produces an image of vastly different quality and contrast than that of a dry fingerprint. Furthermore, skin is not a good absorbing medium; the optical reflectance of human skin lies typically in the range 0.2 to 0.3, and therefore absorption of incident light 10 may be far from total. However, by providing membrane 6 between fingerprint 2 and prism 4, the the frustration of total internal reflection becomes better controlled. Because the thickness of membrane 6 is less than the inter-ridge spacing of fingerprint 2 the membrane 6 conforms substantially to the fingerprint contours and it is apparent that a clear distinction occurs as between those points of contact 14 and non-contact 16 between the membrane 6 and prism 4. Membrane 6, in contact with prism 4, is a more efficient optical coupler than skin would be because of the superior and relatively invariant elastomeric properties of the membrane compared with those of skin; this enables optical contact to be established virtually instantaneously, whereas otherwise this process can take up to ten seconds (Gerbardt et al., 1986). Furthermore, there is no contaminant, such as grease, between the two media. Therefore, the frustrated total internal reflection at point 14 becomes more pronounced and occurs more rapidly than in the prior art apparatus and hence an image of high contrast is achieved rapidly. This contrast may be further enhanced by, for example, constructing membrane 6 from material which is black or dark in colour to ensure efficient absorption at skin-contacting regions 14. Because fingerprint 2 only directly contacts membrane 6 on its upper surface, then prism 4, which contacts only the lower surface of the membrane, will always remain free from dirt or grease contaminants. Hence, build-up of dirt or grease from use by many fingerprints 2 will be restricted to the upper surface of membrane 6, remote from prism 4. In this way, the quality and contrast of the resultant fingerprint image will not become impaired or degraded. Also, it will be apparent that because there will be no contaminant material between membrane 6 and prism 4, then there will be no latent images caused by light-scattering from residual grease.

Similarly, because membrane 6 has a rest position which is spaced apart from the prism 4, then as soon as the fingerprint 2 ceases to apply sufficient pressure to membrane 6 to force it to contact prism 4, then the resultant image will cease because all incident light 10 will be totally internally reflected.

It will be appreciated that since the present apparatus relies on the mere contact or non-contact of membrane 6 upon the surface of prism 4, and that the refractive indices of most elastomers and glasses are similar, then no refractive index-matching materials are required in order to couple the membrane 6 to prism 4.

Furthermore, because it is the elastic membrane 6 and not the skin of fingerprint 2 which contacts prism 4, and the optical and physical properties of the elastic membrane are known and are far more constant than those of skin, for example plasticity and elasticity, then the present apparatus provides for images of a generally more consistent standard and rapidity than prior art devices.

It will also be appreciated that in a fingerprint imaging system which incorporates the present apparatus, it may be convenient for the membrane 6 and frame 8 to form a disposable element, such as a cartridge or disc. By utilising such an arrangement, it is possible to replace the cartridge as and when it is required, or for example, when the membrane 6 is damaged in any way.

Furthermore, it will be apparent to those skilled in the art that the membrane 6 may be formed from any suitable material possessing the required conformal properties herebefore described. The membrane 6 need not be limited to comprising an elastomer.

Additionally, the membrane can be fabricated in the form of an elongate strip with suitable apparatus for tensioning the strip and guide members disposed over the prism.

It will be appreciated that the membrane 6 need not be planar, but may comprise a non-uniform thickness such as having a plurality of relative elevations 9 and depressions 11 on the surface thereof. Such a contoured membrane 6 would tend to obviate "mechanical crosstalk" between those points on the membrane 6 corresponding to adjacent ridges.

## Claims

1. Apparatus for imaging a fingerprint comprising: a light transmitting member having a first major surface at which light transmitted through the light transmitting member may undergo total internal reflection; and a light-absorbing elastic member being arranged adjacent to and spaced from the first major surface for enabling the elastic member, when urged into contact with the light transmitting member by a fingerprint, to conform sympathetically with the fingerprint such that only selected areas of the elastic member, corresponding to fingerprint ridges, contact the first major surface.

2. Apparatus according to Claim 1 wherein the thickness of the elastic member is less than the inter-ridge spacing of the fingerprint.

3. Apparatus according to Claim 1 wherein the elastic member possesses a non-uniform thickness comprising a plurality of relative elevations and depressions.

4. Apparatus according to any one of the preceding claims wherein the elastic member comprises an elastomer.

5. Apparatus according to Claim 4 wherein the elastomer comprises a membrane stretched over a frame.

6. Apparatus according to any one of the preceding claims wherein the elastic member is black in colour.

7. Apparatus according to any one of the preceding claims wherein the light transmitting member is a prism.

2

10

12

4

*Fig.1.*

6

8

*Fig.2.*

2

8

6

5

10

7

4

Fig.3.

2

5

4

6

14

16

Fig.4.

6    9    11

*Fig.5.*

European Patent
Office

**EUROPEAN SEARCH REPORT**

Application Number

EP    92 30 4385

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| Y | US-A-4 525 859 (BOWLES ET AL.)<br>* column 5, line 62 - column 8, line 38 *<br>* figures 1-3 * | 1 | A61B5/117 |
| A | | 2,4,5 | |
| Y | DE-C-691 431 (GRÄPER ET AL.)<br>* the whole document * | 1 | |
| A | | 3,6 | |
| A | EP-A-0 194 783 (FUJITSU LIMITED)<br>* column 10, line 1 - column 11, line 50 *<br>* figures 16,17 * | 1,4 | |
| A | US-A-4 577 345 (ABRAMOV)<br>* abstract *<br>* column 7, line 3 - line 27 *<br>* figures 6,7 * | 1,2,4,5 | |
| A | MACHINE DESIGN.<br>vol. 47, no. 20, 21 August 1975, CLEVELAND US<br>pages 58 - 59; 'Unlocking a door with a fingerprint'<br>* the whole document * | 1,7 | TECHNICAL FIELDS SEARCHED (Int. Cl.5 ) |
| A | FR-A-2 267 735 (AGENCE NATIONALE DE VALORISATION DE LA RECHERCHE)<br>* page 5, line 16 - line 33 *<br>* page 7, line 20 - line 31 *<br>* figures 3,4 * | 3,4 | A61B<br>G06K<br>G07C |

The present search report has been drawn up for all claims

| Place of search | Date of completion of the search | Examiner |
|---|---|---|
| THE HAGUE | 18 AUGUST 1992 | CHEN A.H. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)